# EUROPEAN PATENT APPLICATION

(11) **EP 4 159 245 A1**
(43) Date of publication of application: **05.04.2023**
(21) Application number: 21841292.2
(22) Date of filing: 13.07.2021
(51) Int. Cl.: A61K 51/04, A61K 31/501, C07D 401/12, A61K 101/00, A61K 101/02

(54) **DIAGNOSTIC AGENT FOR PANCREATIC FUNCTION**

(30) Priority: 14.07.2020 JP 2020120505
(71) Applicant: Hamamatsu Photonics K.K., Hamamatsu-shi, Shizuoka 435-8558 (JP)
(72) Inventor: OHBA Hiroyuki, Hamamatsu-shi, Shizuoka 435-8558 (JP); TSUKADA Hideo, Hamamatsu-shi, Shizuoka 435-8558 (JP)
(74) Representative: Grünecker Patent- und Rechtsanwälte PartG mbB
(86) International application number: PCT/JP2021/026345
(87) International publication number: WO 2022/014605

(57) **Abstract**

The present invention relates to a method for diagnosing a pancreatic function, including: a step of administering a diagnostic agent for a pancreatic function which contains a compound represented by General Formula (1-0) as an active component to a subject; a step of detecting the compound (1-0) accumulated in the pancreas; and a step of quantitatively analyzing an amount of compound (1-0) accumulated in the pancreas. 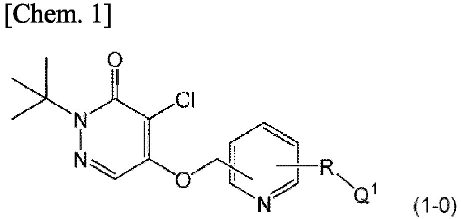 [In General Formula (1-0), R represents -O(CH₂)ₙ-, - O(CH₂)ₙOC₂H₄-, -CH₂O(CH₂)ₙ-, or -CH₂O(CH₂)ₙOC₂H₄-, n represents an integer of 1 to 5, and Q¹ represents F or -OCH₃].

## Description

### Technical Field

The present invention relates to a diagnostic agent for pancreatic function.

### Background Art

The pancreas has: an exocrine function of secreting digestive juice (pancreatic juice) containing amylase that degrades carbohydrates, trypsin that degrades proteins, and lipase that degrades fat; and an endocrine function of secreting hormones such as glucagon and insulin required for glucose metabolism. For this reason, if pancreatic dysfunction occurs, it becomes impossible to produce or absorb energy necessary for survival from food, and the insufficient supply of nutrients to cells leads to functional deterioration of various organs. In addition, even today, when early detection and early treatment of cancer have spread, the 5-year survival rate of pancreatic cancer is still less than 10% because pancreatic cancer is often detected late, so that pancreatic cancer is still among the most intractable cancers.

In the related art, for diagnosis of pancreatic diseases such as acute pancreatitis, chronic pancreatitis, and early pancreatic cancer, for example, diagnostic imaging through X-ray CT and MRI and diagnosis using an ultrasound endoscope have been used (for example, Non Patent Literature 1 and Non Patent Literature 2). On the other hand, Non Patent Literature 3 discloses that uptake of a PET probe ([¹⁸F]FDG) in the pancreas increases in patients with type II diabetes.

### Citation List

### Non Patent Literature

[Non Patent Literature 1] Journal of Abdominal Emergency Medicine, 2008, Vol. 28, pp. 561-571
[Non Patent Literature 2] Journal of the Pancreas, 2009, Vol. 10, pp. 280-283
[Non Patent Literature 3] PLoS ONE, 2019, Vol. 14, e0213202

### Summary of Invention

### Technical Problem

Diagnostic imaging by X-ray CT or MRI in the related art is a diagnosis utilizing information based on structural changes in organs such as atrophy or calcification of the pancreas, so it is impossible to detect changes in biological function prior to structural changes. This is the same for a diagnosis using an ultrasound endoscope. Moreover, the diagnosis using an ultrasound endoscope is also a more invasive examination method. In addition, in the method disclosed in Non Patent Literature 3, a PET examination using [¹⁸F]FDG as a probe is performed, and only an increase in accumulation reflecting inflammation in an organ can be evaluated.

Therefore, an object of the present invention is to provide a diagnostic agent for a pancreatic function that enables early diagnosis of changes in pancreatic function.

### Solution to Problem

The present invention relates to a diagnostic agent for a pancreatic function which contains a compound represented by General Formula (1-0) (hereinafter also referred to as a "compound (1-0)") as an active component.

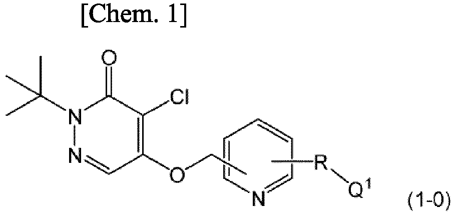

In General Formula (1-0), R represents -O(CH₂)ₙ-, - O(CH₂)ₙOC₂H₄-, -CH₂O(CH₂)ₙ-, or -CH₂O(CH₂)ₙOC₂H₄-, n represents an integer of 1 to 5, and Q¹ represents F or -OCH₃.

It is known that the compound (1-0) can be used for detecting mitochondrial complex I (hereinafter also referred to as "MC-I"). The diagnostic agent for a pancreatic function according to the present invention accumulates in the pancreas and the amount of the agent accumulated is proportional to an MC-I activity of the pancreas, and therefore the agent is suitably used for diagnosing a pancreatic function. In addition, as shown in the examples to be described below, a decrease in MC-I activity of the pancreas precedes deterioration in biochemical indicators (for example, an increase in blood glucose concentration). Accordingly, it is possible to diagnose changes in pancreatic function in an early stage using the diagnostic agent according to the present invention.

In the above-described diagnostic agent, Q¹ may be ¹⁸F or - O¹¹CH₃. Accordingly, the compound can release positrons. The positrons released from the above-described compound immediately bind to electrons to release a γ-ray (annihilation radiation). By measuring these γ-rays with a device used for positron emission tomography (PET method), the above-described compound accumulated in the pancreas can be imaged quantitatively over time. That is, the compound can also be used as a labeled compound for the PET method.

The present invention can also be regarded as a method for diagnosing a pancreatic function, including: a step of administering the above-described diagnostic agent to a subject; a step of detecting the compound (1-0) accumulated in the pancreas; and a step of quantitatively analyzing an amount of compound (1-0) accumulated in the pancreas.

The present invention can also be regarded as a compound represented by General Formula (1-0) for use in diagnosis of a pancreatic function. The present invention can also be regarded as use of a compound represented by General Formula (1-0) in manufacture of a diagnostic agent for a pancreatic function.

### Advantageous Effects of Invention

According to the present invention, it is possible to provide a diagnostic agent for a pancreatic function that enables early diagnosis of changes in pancreatic function.

### Brief Description of Drawings

FIG. 1 is a graph illustrating accumulation of [¹⁸F]BCPP-BF in the pancreas by plotting the amount (SUV) of radioactivity accumulated measured through a PET measurement method against the amount (SUV) of radioactivity accumulated measured through a dissection method.
FIG. 2 shows graphs illustrating results of obtained by measuring uptake (SUV) of [¹⁸F]BCPP-BF in the pancreas (FIG. 2(A)), the weight of the pancreas (FIG. 2(B)), and the blood glucose concentration (FIG. 2(C)) for each of week-old lean rats and fatty rats.
FIG. 3 shows graphs illustrating relationships between uptake (SUV) of [¹⁸F]BCPP-BF in the pancreas and the blood glucose concentration or the blood neutral fat concentration.
FIG. 4(A) is a graph illustrating the number of insulin-positive cells per unit area of the pancreas of 5-week-old and 16-week-old rats. FIG. 4(B) is a graph illustrating a relationship between uptake (SUV) of [¹⁸F]BCPP-BF in the pancreas of 16-week-old rats (lean and fatty rats) and the number of insulin-positive cells per unit area.

### Description of Embodiment

Hereinafter, an embodiment of the present invention will be described in detail. However, the present invention is not limited to the following embodiment.

The diagnostic agent for a pancreatic function according to the present embodiment contains a compound represented by General Formula (1-0) as an active component.

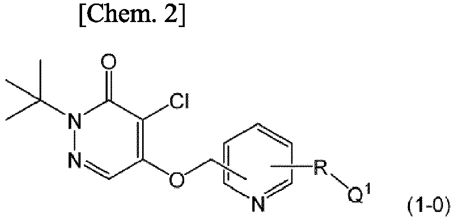

In the compound (1-0), R represents -O(CH₂)ₙ-, -O(CH₂)ₙOC₂H₄-, -CH₂O(CH₂)ₙ-, or -CH₂O(CH₂)ₙOC₂H₄-. R is preferably -O(CH₂)ₙ- or - O(CH₂)ₙOC₂H₄- and more preferably -O(CH₂)ₙ-.

In the compound (1-0), n is an integer of 1 to 5, preferably an integer of 2 to 5, more preferably an integer of 3 to 5, and still more preferably 4.

In the compound (1-0), Q¹ is F or -OCH₃ and preferably ¹⁸F or - O¹¹CH₃. Since the compound (1-0) in which Q¹ is ¹⁸F or -O¹¹CH₃ can release positrons, it is suitable as a labeled compound (PET probe) for use in a PET method. In addition, in a case where Q¹ is -O¹¹CH₃, the half-life is as short as 20 minutes, so it is also possible to perform measurement multiple times on the same subject in one day. In a case where Q¹ is ¹⁸F, the half-life is 110 minutes which is longer than that of -O¹¹CH₃, so it is possible to lengthen the time for one measurement.

A binding site of R and a binding site of -OCH₂- which binds to a pyridazine ring are not particularly limited, but the binding site of -OCH₂- binding to a pyridazine ring is preferably at position 5 of the pyridine ring and the binding site of R is preferably at position 2 of the pyridine ring. A compound represented by General Formula (1-0') shown below (hereinafter also referred to as a "compound (1-0')") has a structural formula in which the binding site of -OCH₂- binding to a pyridazine ring is at position 5 of the pyridine ring and the binding site of R is at position 2 of the pyridine ring.

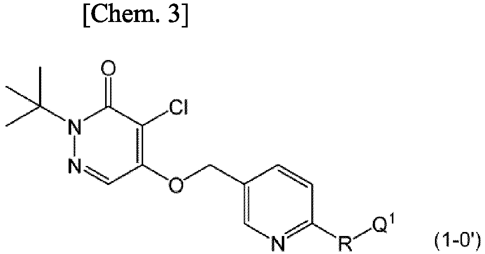

In General Formula (1-0'), R, n, and Q¹ are synonymous with R, n, and Q¹ in General Formula (1-0).

The compound (1-0) is preferably a compound represented by General Formula (1-0") (hereinafter also referred to as a "compound (1-0")") and more preferably a compound represented by Formula (1) (hereinafter also referred to as a "compound (1)") because it is more suitably used for diagnosing a pancreatic function.

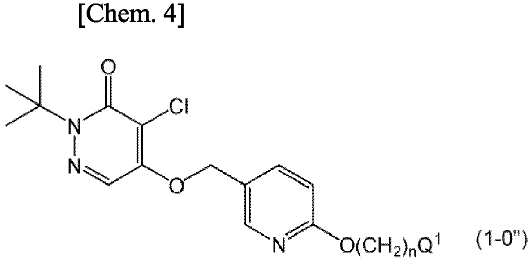

In General Formula (1-0"), n and Q¹ are synonymous with n and Q¹ in General Formula (1-0).

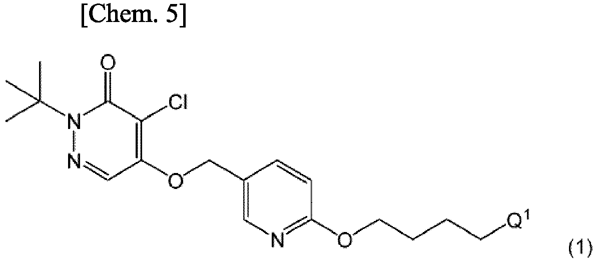

In Formula (1), Q¹ is synonymous with Q¹ in General Formula (1-0).

The compound (1-0) can be synthesized, for example, from a corresponding precursor. The same applies to the compound (1-0'), the compound (1-0"), and the compound (1).

Examples of precursors corresponding to the compound (1-0) include a compound represented by General Formula (2-0) below (hereinafter also referred to as a "compound (2-0)"). Examples of precursors corresponding to the compound (1-0'), the compound (1-0"), and the compound (1) include compounds in which, in the compound (2-0), R, the binding site of R, and the binding site of -OCH₂- binding to a pyridazine ring in a pyridine ring are the same as those in the compound (1-0'), the compound (1-0"), and the compound (1).

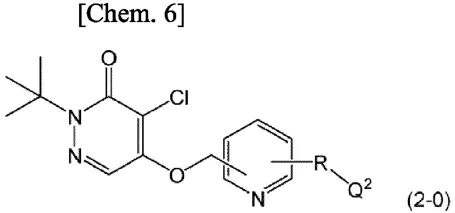

In General Formula (2-0), R is synonymous with R in General Formula (1-0). Q² represents a removable substituent (a substituted sulfonyloxy group, a halogen atom, or a hydroxyl group).

Examples of substituted sulfonyloxy groups include a tosyloxy group (-OTs), a methanesulfonyloxy group (-OMs), a trifluoromethanesulfonyloxy group (-OTf), and a nitrobenzenesulfonyloxy group (-ONs), and -OTs is preferably used.

Examples of halogen atoms include fluorine, chlorine, bromine, and iodine.

A precursor can be synthesized through, for example, a method disclosed in PCT International Publication No. WO2014/30709.

Since the compound (1-0) accumulates in the pancreas specifically for MC-1, the accumulation amount changes in correlation with the degree of a pancreatic function. That is, the accumulation amount of compound (1-0) decreases when the pancreatic function deteriorates, and the accumulation amount of compound (1-0) increases when the pancreatic function is enhanced. Accordingly, the diagnostic agent according to the present embodiment is suitably used for diagnosing a pancreatic function through measurement of the accumulation amount of compound (1-0).

The measurement of the accumulation amount of compound (1-0) is not limited to this, and can be carried out such that, for example, the compound (1-0) is bound to a fluorescent dye or the like or labeled with a single photon nuclide (such as ¹²³I, ^{99m}Tc) or a positron nuclide to form a labeled compound, and the label is detected. The positron labeling can be performed, for example, by setting Q¹ of the compound (1-0) to - O¹¹CH₃ or ¹⁸F. In the case of the positron labeling, biodistribution of the compound (1-0) can be quantitatively imaged over time by measuring annihilation radiation with a device used for a PET method.

The diagnostic agent according to the present embodiment can be produced, for example, by dissolving the compound (1-0) in an arbitrary buffer solution. In this case, the diagnostic agent according to the present embodiment may be provided as a solution and contain other components such as a surfactant, a preservative, and a stabilizer in addition to the buffer component.

A method for diagnosing a pancreatic function according to the present embodiment includes: a step of administering the above-described diagnostic agent to a subject; a step of detecting the compound (1-0) accumulated in the pancreas; and a step of quantitatively analyzing an amount of compound (1-0) accumulated in the pancreas.

Examples of subjects include humans, monkeys, mice, and rats, but are not limited thereto.

A method for administering a diagnostic agent to a subject is not particularly limited as long as the compound (1-0) reaches the pancreas, but is usually intravenous administration.

The dose of a diagnostic agent is not particularly limited as long as it is a sufficient dose to detect the compound (1-0) in the pancreas, but may be appropriately set according to subjects to which it is administered and methods for detecting the compound (1-0). For example, in a case where the compound (1-0) is detected with a device used for a PET method using a diagnostic agent containing a compound (1-0) in which Q¹ is ¹⁸F or -O¹¹CH₃, the dose (hereinafter also referred to as an "amount of radioactivity administered") of the diagnostic agent may be 1 MBq/kg body weight to 1,000 MBq/kg body weight. The specific radioactivity of the compound (1-0) may be 10 to 10,000 GBq/µmol. In addition, the amount of radioactivity administered of a diagnostic agent depends on the sensitivity of a PET camera used and the volume of an individual subject, but about 200 to 500 MBq/kg body weight thereof is administered as 0.1 to 0.5 mL of a physiological saline solution for rodents (mice and rats). In a case of non-human primates (monkeys), 40 to 200 MBq/kg body weight thereof is administered as 0.5 to 2 mL of physiological saline solution, and in a case of humans, 2 to 10 MBq/kg body weight thereof is administered as 1 to 5 mL of a physiological saline solution.

The method for detecting the compound (1-0) accumulated in the pancreas is not particularly limited, and can be carried out according to well-known methods. For example, in a case where a diagnostic agent containing a compound (1-0) in which Q¹ is ¹⁸F or -O¹¹CH₃ is used, the compound (1-0) can be detected through a PET method. The measurement method in the PET method is not particularly limited, and can be carried out according to well-known methods. In addition, for example, as a measurement method in the PET method, dynamic measurement may be performed for 60 minutes immediately after administering a diagnostic agent, or PET measurement may be performed for 10 to 20 minutes after administering a diagnostic agent and waiting for 30 to 40 minutes to allow sufficient accumulation of the compound (1-0) in the pancreas.

A method for quantitatively analyzing the amount of compound (1-0) accumulated in the pancreas is not particularly limited, and can be carried out according to well-known methods. For example, the following method is exemplified. First, an integrated image of a compound (1-0) obtained through a PET method and a morphological image of the pancreas obtained through CT measurement or the like are superimposed to identify a PET image of the pancreas. Next, a region of interest on the PET image of the pancreas is set, and a value normalized by the amount of radioactivity administered and the body weight of an individual subject is defined as an amount of compound (1-0) accumulated in the pancreas. In addition, an image obtained by a PET method in which a probe capable of detecting the pancreas is used may be used instead of the morphological image of the pancreas.

The diagnostic method according to the present embodiment may further include a step of comparing the quantitatively analyzed accumulation amount of compound (1-0) with a reference value to diagnose a pancreatic function.

The reference value may be appropriately set depending on the purpose of diagnosis. For example, in a case where the diagnostic method according to the present embodiment is carried out in a group medical examination, the reference value may be a normal range previously determined from distribution of the accumulation amount of compound (1-0) in a plurality of the same kinds of subjects. In this case, whether or not the pancreatic function of a specific subject is normal can be diagnosed depending on whether or not the quantitatively analyzed value of the accumulation amount of the specific subject is within the normal range.

In addition, in a case where the diagnostic method according to the present embodiment is performed on subjects suffering from, for example, symptoms (for example, diabetes, lipid metabolism disorder, chronic pancreatitis, acute pancreatitis, and pancreatic cancer) which cause pancreatic dysfunction for follow-up observation of symptoms, confirmation of treatment effects, prognosis prediction, and the like, the reference value may be a measurement result of the accumulated amount of compound (1-0) of the subjects at a certain point in time (for example, at a point in time of being healthy, during initial diagnosis, at the beginning of treatment, or at the end of treatment).

The above-described present invention can also be regarded as a compound represented by General Formula (1-0) for use in diagnosis of a pancreatic function. The diagnosis of a pancreatic function can be carried out through a method exemplified in the above-described method for diagnosing a pancreatic function.

### Example

Hereinafter, the present invention will be described in more detail based on examples. However, the present invention is not limited to thereto.

### [Test Example 1: Synthesis of PET Probe]

[¹⁸F]BCPP-BF represented by the following formula was synthesized through the method disclosed in Non Patent Literature (J. Labeled Comp. Radiopharm., 2013, Vol. 56, No. 11, pp. 553-561). The final product obtained had a radiochemical purity of 100.0% and a specific radioactivity of 74.5 GBq/µmol.

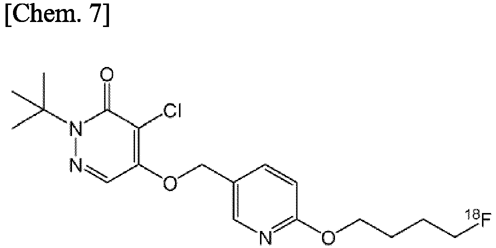

In addition, a probe (D-[¹¹C]MT) that recognizes amino acid transporter (LAT-1) highly expressed in pancreases of small animals was prepared to specify the position of the pancreas. D-[¹¹C]MT was synthesized through a method disclosed in Example 1 of PCT International Publication No. WO2005/115971. The final product obtained had a radiochemical purity of 100.0% and a specific radioactivity of 56.4 GBq/µmol.

### [Test Example 2: Evaluation of Pancreatic Function in Type II Diabetes Model Rats]

### (Type II Diabetes Model Rats)

Male Zucker Leprfa/Leprfa rats (hereinafter also referred to as "fatty rats") that develop pathological conditions similar to type II diabetes of human adults and male Zucker Leprfa/+ rats (hereinafter also referred to as "lean rats") as controls thereof were purchased from Charles River Laboratories Japan, Inc., and used for PET measurement at 5, 8, 16, and 26 weeks of age.

### (PET Measurement)

The rats were anesthetized with isoflurane and fixed in a gantry of an animal PET camera (SHR-38000, manufactured by Hamamatsu Photonics K.K.). After 15-minute transmission measurement was carried out for absorption correction, about 20 MBq/0.5 mL of D-[¹¹C]MT was administered to the rats via the tail vein and 60-minute emission measurement was carried out. Subsequently, about 20 MBq/0.5 mL [¹⁸F]BCPP-BF was administered to the rats via the tail vein, and 60-minute emission measurement was carried out. After the completion of the PET measurement, a region of interest was set on the pancreas identified from an accumulated PET image 40 to 60 minutes after the administration of D-[¹¹C]MT, and the amount of [¹⁸F]BCPP-BF accumulated in the region of interest was calculated. Subsequently, the calculated accumulation amount was normalized by the amount of radioactivity administered and the body weight of each individual and defined as an amount (amount (SUV) of radioactivity administered) of [¹⁸F]BCPP-BF accumulated in the pancreas.

### (Measurement of Amount (SUV) of Radioactivity Accumulated Through Dissection Method)

The pancreas was excised from each rat immediately after the completion of the PET measurement, and the weight of the excised pancreas was measured. Subsequently, the radioactivity of the excised pancreas was measured using a gamma counter (manufactured by PerkinElmer Inc., 1480 WIZARD). The measured radioactivity was normalized by the body weight of each rat, the weight of the pancreas, and the amount of radioactivity administered to calculate an amount (SUV) of radioactivity accumulated through a dissection method.

### (Measurement of Blood Glucose Concentration and Blood Neutral Fat Concentration)

Blood was collected from each rat immediately after the PET measurement, and the blood glucose concentration and the blood neutral fat concentration were measured. The blood glucose concentration and the blood neutral fat concentration were measured using a biochemical automatic analyzer (7180 manufactured by Hitachi High-Tech Corporation.).

### (Measurement of Number of Insulin-Positive Cells)

The excised pancreas after measuring its weight and radioactivity was used to measure the number of insulin-positive cells (cells/µm²) through immunostaining. Specifically, a pancreatic tissue slice was stained with an anti-insulin monoclonal antibody (manufactured by Nichirei Corporation, K36aC10) as a primary antibody, Histofine Simple Stain (manufactured by Nichirei Corporation, MAX-PO (M)) as a secondary antibody, and DAB (manufactured by Dojindo Laboratories, D006) as a color developer, and 5 pancreatic islets were randomly selected from the tissue slice and imaged at 100× with an optical microscope (manufactured by Olympus Corporation, BX41) and a microscope camera (manufactured by Olympus Corporation, DP22), and the number of insulin-positive cells (cells/µm²) was measured using dedicated image software (manufactured by Olympus Corporation, Cell Sens v1.15).

### (Results)

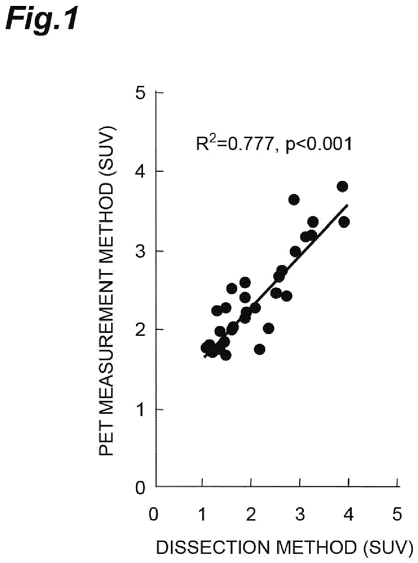

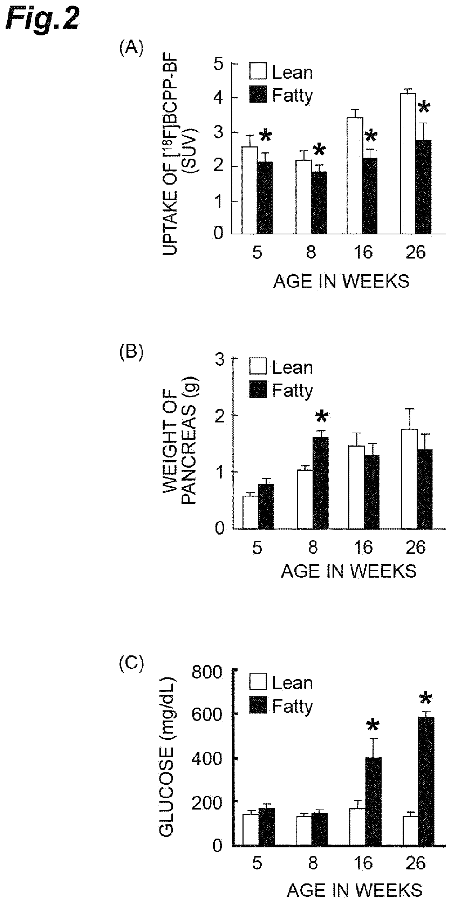

FIG. 1 is a graph illustrating accumulation of [¹⁸F]BCPP-BF in the pancreas by plotting the amount (SUV) of radioactivity accumulated measured through a PET measurement method against the amount (SUV) of radioactivity accumulated measured through a dissection method. As shown in FIG. 1, the SUV measured through the PET measurement method and the SUV measured through the dissection method showed a favorable correlation (R²=0.777, p<0.001). It can be seen that the measurement of MC-I in the pancreas can be carried out non-invasively using [¹⁸F]BCPP-BF. Hereinafter, uptake (SUV) of [¹⁸F]BCPP-BF in the pancreas shown in FIGS. 2 to 4 is an amount (SUV) of radioactivity accumulated which is measured through the PET measurement method.

FIG. 2 shows graphs illustrating results of obtained by measuring uptake (SUV) of [¹⁸F]BCPP-BF in the pancreas (FIG. 2(A)), the weight of the pancreas (FIG. 2(B)), and the blood glucose concentration (FIG. 2(C)) for each of week-old lean rats and fatty rats. In FIG. 2, "*" indicates that the difference between the fatty rat data and the lean rat data was statistically significant (p < 0.05).

As shown in FIG. 2(B), in the fatty rats, enlargement of the pancreas at 8 weeks of age was observed, and atrophy of the pancreas was observed thereafter. In addition, as shown in FIG. 2(C), in the fatty rats, abnormal blood glucose concentration was detected after 16 weeks of age. On the other hand, as shown in FIG. 2(A), in the fatty rats, uptake (SUV) of [¹⁸F]BCPP-BF in the pancreas decreased after 5 weeks of age. The decrease in uptake (SUV) of [¹⁸F]BCPP-BF in the pancreas means a decrease in MC-I activity. It can be seen from these results that the decrease in the pancreatic function can be detected by the PET measurement method using [¹⁸F]BCPP-BF before an abnormal blood glucose concentration is detected (here, at 5 and 8 weeks of age).

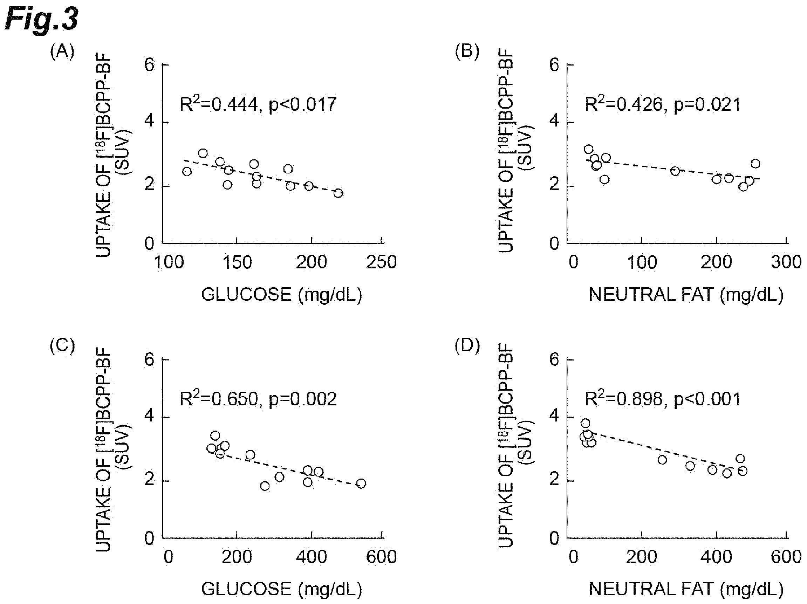

FIG. 3 shows graphs illustrating relationships between uptake (SUV) of [¹⁸F]BCPP-BF in the pancreas and the blood glucose concentration or the blood neutral fat concentration. FIG. 3(A) is a graph in which the blood glucose concentration and uptake (SUV) of [¹⁸F]BCPP-BF in the pancreas of 5-week-old rats (lean and fatty rats) are plotted. FIG. 3(B) is a graph in which the blood neutral fat concentration and uptake (SUV) of [¹⁸F]BCPP-BF in the pancreas of 5-week-old rats (lean and fatty rats) are plotted. FIG. 3(C) is a graph in which the blood glucose concentration and uptake (SUV) of [¹⁸F]BCPP-BF in the pancreas of 16-week-old rats (lean and fatty rats) are plotted. FIG. 3(D) is a graph in which the blood neutral fat concentration and uptake (SUV) of [¹⁸F]BCPP-BF in the pancreas of 16-week-old rats (lean and fatty rats) are plotted.

It has been reported that a high blood glucose concentration or a high neutral fat concentration induces dysfunction in the pancreas (for example, FEBS J., 2013, Vol. 280, pp. 1039-1050, Endocr Rev., 2008, Vol. 29, pp. 351-366, and Kidney Int., 2006, Vol. 70, pp. 1560-1566). As shown in FIG. 3, this phenomenon could be detected as an impairment of an MC-I activity using [¹⁸F]BCPP-BF.

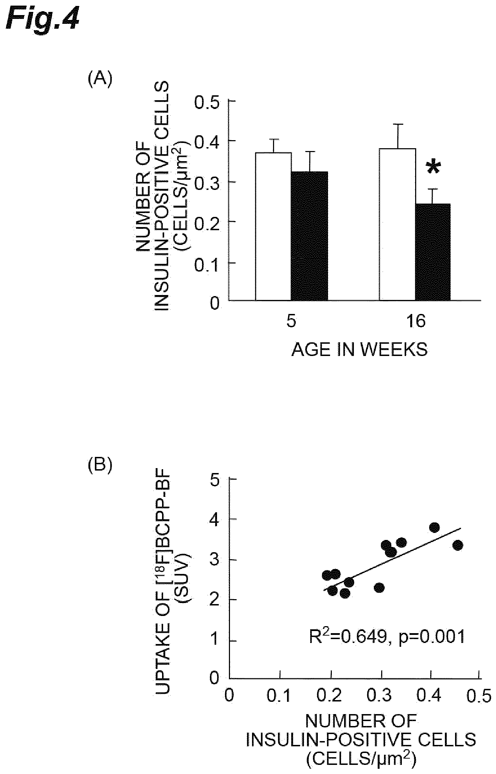

FIG. 4(A) is a graph illustrating the number of insulin-positive cells per unit area of the pancreas of 5-week-old and 16-week-old rats. In FIG. 4(A), the white bars show results of the lean rats, and the black bars show results of the fatty rats. FIG. 4(B) is a graph illustrating a relationship between uptake (SUV) of [¹⁸F]BCPP-BF in the pancreas of 16-week-old rats (lean and fatty rats) and the number of insulin-positive cells per unit area.

As shown in FIG. 4(A), as a result of counting the number of insulin-positive cells which is an existing indicator of the pancreatic function through an immunostaining method, no significant difference was observed between the lean and fatty rats at 5 weeks of age, and a significant decrease was detected in the fatty rat at 16 weeks of age. As shown in FIG. 2(A), in the PET measurement method using [¹⁸F]BCPP-BF, the decrease in MC-I activity of the pancreas could be detected in the fatty rat at 5 weeks of age.

As shown in FIG. 4(B), the MC-I activity (the uptake (SUV) of [¹⁸F]BCPP-BF in the pancreas) measured through the PET measurement method using [¹⁸F]BCPP-BF in the 16-week-old rats (lean and fatty rats) showed a significant positive correlation with the number of insulin-positive cells per unit area (R²=0.649, p=0.001).

## Claims

1. A method for diagnosing a pancreatic function, comprising:
a step of administering a diagnostic agent for a pancreatic function which contains a compound represented by General Formula (1-0) as an active component to a subject;
a step of detecting the compound (1-0) accumulated in the pancreas; and
a step of quantitatively analyzing an amount of compound (1-0) accumulated in the pancreas, 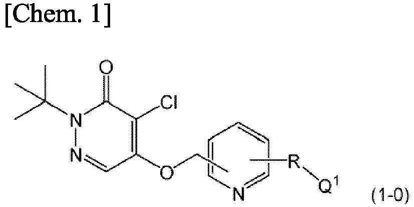 [in General Formula (1-0), R represents -O(CH₂)ₙ-, - O(CH₂)ₙOC₂H₄-, -CH₂O(CH₂)ₙ-, or -CH₂O(CH₂)ₙOC₂H₄-, n represents an integer of 1 to 5, and Q¹ represents F or -OCH₃].

2. The diagnostic method according to claim 1,
wherein the active component is a compound represented by General Formula (1-0'), 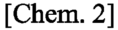 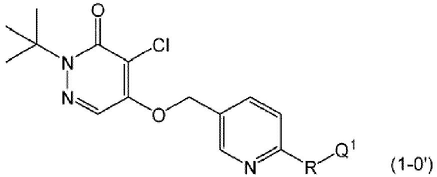 [in General Formula (1-0'), R, n, and Q¹ are synonymous with R, n, and Q¹ in General Formula (1-0)].

3. The diagnostic method according to claim 1,
wherein the active component is a compound represented by General Formula (1-0"), 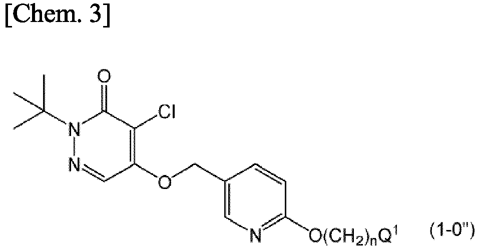 [in General Formula (1-0"), n and Q¹ are synonymous with n and Q¹ in General Formula (1-0)].

4. The diagnostic method according to claim 1,
wherein the active component is a compound represented by Formula (1) below, 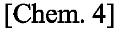 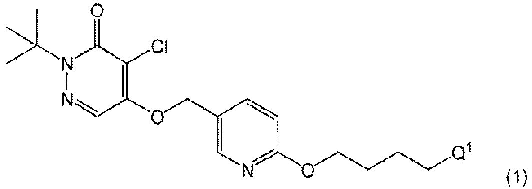 [in Formula (1), Q¹ is synonymous with Q¹ in General Formula (1-0)].

5. The diagnostic method according to any one of claims 1 to 4,
wherein Q¹ is ¹⁸F or -O¹¹CH₃.

6. A compound represented by General Formula (1-0) for use in the method for diagnosing a pancreatic function according to any one of claims 1 to 5, 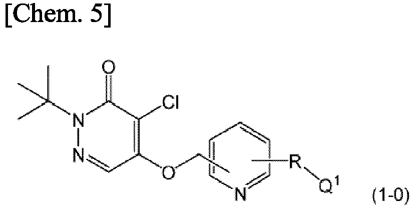 [in General Formula (1-0), R represents -O(CH₂)ₙ-, - O(CH₂)ₙOC₂H₄-, -CH₂O(CH₂)ₙ-, or -CH₂O(CH₂)ₙOC₂H₄-, n represents an integer of 1 to 5, and Q¹ represents F or -OCH₃].

7. Use of a compound represented by General Formula (1-0) in manufacture of a diagnostic agent for a pancreatic function, 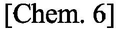 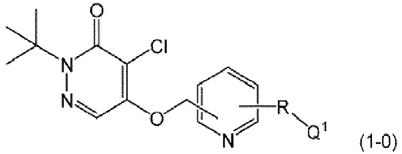 [in General Formula (1-0), R represents -O(CH₂)ₙ-, - O(CH₂)ₙOC₂H₄-, -CH₂O(CH₂)ₙ-, or -CH₂O(CH₂)ₙOC₂H₄-, n represents an integer of 1 to 5, and Q¹ represents F or -OCH₃].
